**Europäisches Patentamt**

**European Patent Office**

**Office européen des brevets**

(19)

(11) Veröffentlichungsnummer : **0 210 160 B2**

# (12) NEUE EUROPÄISCHE PATENTSCHRIFT

(45) Veröffentlichungstag der neuen Patentschrift :
**15.07.92 Patentblatt 92/29**

(51) Int. Cl.⁵ : **A61M 5/178, A61M 5/31,**
**A61M 31/00, A61M 35/00**

(21) Anmeldenummer : **86890174.5**

(22) Anmeldetag : **12.06.86**

(54) **Vorrichtung zur Applikation eines Gewebeklebstoffes.**

(30) Priorität : **20.06.85 AT 1838/85**

(43) Veröffentlichungstag der Anmeldung :
**28.01.87 Patentblatt 87/05**

(45) Bekanntmachung des Hinweises auf die
Patenterteilung :
**03.05.89 Patentblatt 89/18**

(45) Bekanntmachung des Hinweises auf die
Entscheidung über den Einspruch :
**15.07.92 Patentblatt 92/29**

(84) Benannte Vertragsstaaten :
**AT BE CH DE FR GB IT LI NL SE**

(56) Entgegenhaltungen :
**AT-B- 9 324**
**AT-B- 366 916**
**AT-B- 379 311**
**DE-A- 1 965 990**
**DE-B- 2 012 607**
**DE-C- 419 869**
**FR-A- 1 051 010**
**US-A- 3 223 083**

(56) Entgegenhaltungen :
**Gastroenterology, vol. 77, 1979, Seiten 642 bis
646, W. G. Linscheer et al: "Control of Upper
Gatrsointestinal Hemorrhage byEndoscopis
Spraying of Clotting Factors".**
**Die Medizinische Welt, Band 36, 1985, Seiten
769 bis 776, H. Redl et al: "Vergleich zweier
Fibrinkleber"**

(73) Patentinhaber : **IMMUNO Aktiengesellschaft
für chemisch-medizinische Produkte
Industriestrasse 72
A-1220 Wien (AT)**

(72) Erfinder : **Eibl, Johann, Dr.
Gustav Tschermakgasse 2
A-1180 Wien (AT)**
Erfinder : **Seelich, Thomas, Dr.
Gerhardusgasse 10/10
A-1200 Wien (AT)**
Erfinder : **Redl, Heinz, Doz. Dr.
Windmühlgasse 7
A-1060 Wien (AT)**
Erfinder : **Habison, Georg, Dr.
Taubstummengasse 15/12
A-1040 Wien (AT)**

(74) Vertreter : **Wolfram, Gustav, Dipl.-Ing.
Patentanwälte Sonn, Pawloy, Weinzinger &
Wolfram Riemergasse 14
A-1010 Wien (AT)**

EP 0 210 160 B2

**Beschreibung**

Die Erfindung betrifft eine Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen mit Thrombin in situ verfestigt wird, mit zwei mit gemeinsam betätigbaren Kolben versehenen Spritzenkörpern, wobei der eine die Proteinkomponente und der andere die Thrombinkomponente beinhaltet, und auf die Spritzenkörper ein Verbindungskopf aufsetzbar ist, der für jede der zu applizierenden Komponenten und gegebenenfalls für ein medizinisches Treibgas einen eigenen Förderkanal aufweist.

Vorrichtungen dieser Art werden in der AT-B-366 916 und AT-B-379 311 beschrieben. Als Proteinkomponente wird eine Faktor XIII und Fibrinogen enthaltende Lösung (Gewebeklebstoff), und als Thrombinkomponente eine wässerige Thrombinlösung verwendet. Diese Komponenten werden in einer auf den Verbindungskopf aufgesetzten Mischkanüle vermischt und auf die zu behandelnde bzw. zu schützende Wundstelle aufgebracht.

Bei Durchführung von Gewebeklebungen bzw. -behandlungen werden üblicherweise die Proteinlösung und die Thrombinlösung durch Auflösen von Lyophilisaten erhalten. Dabei kann sich insbesondere bei einem chirurgischen Notfall die Schwierigkeit ergeben, daß die Bereitung der Proteinlösung infolge der schweren Löslichkeit dieser Komponente eine unerwünscht lange Zeit in Anspruch nimmt. Es ist zwar möglich. die Auflösungsdauer der Proteinkomponente durch Verwendung einer größeren Menge von Lösungsmittel zu verkürzen, jedoch wird bei der Mischung der Proteinlösung mit der Thrombinlösung im Volumverhältnis von 1:1, wie bisher üblich, auch die clottierende Mischung so stark verdünnt, daß eine signifikante Verringerung der Reißfestigkeit der Klebung als Folge auftritt.

Die Erfindung bezweckt die Vermeidung dieser Schwierigkeit und stellt sich die Aufgabe, die bekannte Vorrichtung so auszubilden, daß bei der Bereitung der Proteinlösung aus einem Lyophilisat die Auflösungsdauer verkürzt werden kann, ohne eine Verringerung der Reißfestigkeit der Klebung in Kauf nehmen zu müssen.

Gemäß der Erfindung wird die gestellte Aufgabe dadurch gelöst, daß die Spritzenkörper als Doppelkammerspritzenkörper ausgebildet sind, wobei in den mündungsseitigen Kammern die Proteinkomponente und die Thrombinkomponente in jeweils lyophilisierter Form und in den anderen Kammern das Lösungsmittel untergebracht sind und wobei auf die Anschlußstücke der Spritzenkörper ein Verbindungskopf aufsetzbar ist, der für jede der zu applizierenden Komponenten und gegebenenfalls für ein medizinisches Treibgas einen eigenen Förderkanal aufweist, wobei bei gleicher wirksamer Hubhöhe der Spritzenkörper der die Proteinlösung beinhaltende Spritzenkörper eine um das Zwei- bis Neunfache größere Querschnittsfläche als der die Thrombinlösung beinhaltende Spritzenkörper aufweist, so daß sich die Volumina der Spritzenkörper ebenfalls wie 2 : 1 bis 9 : 1 verhalten.

Während bisher die für Gewebeklebungen verwendeten Proteinlösungen einen Fibrinogengehalt von etwa 10% (100 mg/ml) oder mehr aufgewiesen haben, können erfindungsgemäß verdünnte Proteinlösungen mit einem Fibrinogengehalt von 2 bis 7% eingesetzt werden oder es können bei Verwendung von Proteinlösungen mit einem Gehalt an Fibrinogen bis 12% höhere Festigkeiten der Klebungen erreicht werden.

Die Erfindung beruht auf dem Prinzip, daß ein günstiges Verhältnis zwischen der notwendigen Rekonstitutionszeit des Proteinlyophilisates und der Festigkeit der Klebungen erreicht wird, wenn das bisher übliche Mischungsverhältnis der Proteinlösung und der Thrombinlösung von 1:1 derart abgeändert wird, daß die Proteinkomponente in einem relativ größeren Volumen gelöst wird und die sich daraus ergebende niedrigere Proteinkonzentration durch Lösung der Thrombinkomponente in einem relativ kleineren Volumen nach Mischen der beiden Komponenten kompensiert wird.

Werden andererseits Klebungen von besonders hoher Festigkeit gefordert, so kann erfindungsgemäß eine Erhöhung der Konzentration der Kleberproteine nach Mischung der beiden Komponenten erreicht werden, ohne daß eine Verlängerung der Rekonstitutionszeit in Kauf genommen werden muß. Eine annähernd genaue Mischung der beiden Komponenten in einem von 1 abweichenden Verhältnis wäre aus zwei getrennten Spritzen zu schwierig und wird erst durch die erfindungsgemäße Doppelspritze möglich.

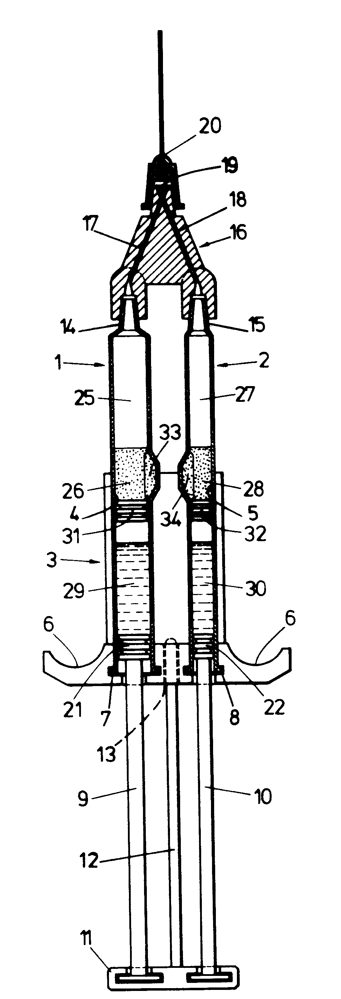

Die erfindungsgemäße Vorrichtung ist an einem Ausführungsbeispiel in der Zeichnung, die eine Seitenansicht teilweise geschnitten darstellt, näher erläutert.

Mit 1 und 2 sind die als Doppelkammerspritzen ausgebildeten Spritzenkörper bezeichnet. Sie sind gemeinsam in U-förmig gestalteten Rinnen 4, 5 einer Halteeinrichtung 3 eingesetzt. Am Ende der Halteeinrichtung sind Fingergriffe 6 vorgesehen, in die die Flanschenden 7 und 8 der Spritzenkörper ragen, so daß diese in Richtung ihrer jeweiligen Längsachse fixiert sind. In den Spritzenkörpern sind Kolben 21, 22 geführt, deren Kolbenstangen 9 und 10 nach außen ragen. Sie weisen eine gemeinsame Betätigungseinrichtung 11 auf.

Mit 12 ist eine Führungsstange bezeichnet, die eine Bohrung 13 der Halteeinrichtung durchsetzt. Auf die Konusse 14, 15 der Spritzenkörper 1, 2 ist ein Verbindungskopf 16 aufsetzbar, der Förderkanäle 17, 18 auf-

2

weist, die an der Stirnseite 19 des sockelförmigen Verbindungskopfes enden. Auf diesen Sockel ist die Mischkanüle 20 aufgesetzt.

In der mündungsseitigen Kammer 25 des Spritzenkörpers 1 sind das lyophilisierte Kleberprotein, welches mit 26 bezeichnet ist, und in der mündungsseitigen Kammer 27 des Spritzenkörpers 2 Thrombin, das mit 28 bezeichnet ist, eingefüllt.

In den jeweils von der Mündungsseite abliegenden Kammern 29 und 30 befindet sich das Lösungsmittel, u.zw. in der Kammer 29 eine Aprotinin enthaltende Lösung und in der Kammer 30 eine Calciumchlorid enthaltende Lösung. Die Kammern 25 und 29 sind durch einen schwimmenden Kolben 31 und die Kammern 27 und 30 durch einen schwimmenden Kolben 32 voneinander getrennt. Mit 33 und 34 sind die Überbrückungsleitungen bezeichnet.

Auf den Kolbenstangen 9 und 10 sind die Kolben 21 und 22 befestigt. Nach ihrer Betätigung wird die Flüssigkeit aus den Kammern 29, 30 durch die Überbrückungsleitungen in die Kammern 25 und 28 gedrückt und die zur Applikation erforderlichen Lösungen bereitet.

Das Querschnittsverhältnis des Spritzenkörpers 1 zum Spritzenkörper 2 beträgt 3 : 1, so daß sich auch die Volumina wie 3 : 1 verhalten.

In Vergleichsversuchen, worin einerseits eine Kleberproteinlösung in herkömmlicher Weise unter Verwendung der bekannten Vorrichtung gemäß der AT-B - 366.916 mit einer Thrombinlösung im Volumsverhältnis 1 : 1 gemischt wurde und andererseits Kleberproteinlösungen mit verschiedenen Fibrinogengehalten unter Anwendung der erfindungsgemäßen Arbeitsweise bzw. Vorrichtungen mit Thrombinlösungen in verschiedenen Mischungsverhältnissen gemischt und die Eigenschaften der verfestigten Klebstoffe untersucht worden sind, wurde herausgefunden, daß es gelingt, ohne Verschlechterung der Reißfestigkeit die benötigte Lösungszeit zu verkürzen, oder umgekehrt, ohne eine verlängerte Lösungszeit in Kauf nehmen zu müssen, die Reißfestigkeit zu erhöhen.

Das erfindungsgemäße Applikationsgerät ist auch für die Anwendung hoher Thrombinkonzentrationen geeignet. In der chirurgischen Praxis ist die Verwendung hoher Thrombinkonzentrationen, insbesondere zur Stillung von Blutungen, von großer Bedeutung.

**Patentansprüche**

1. Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen mit Thrombin in situ verfestigt wird, mit zwei in Anschlußstücke endigenden und mit gemeinsam betätigbaren Kolben (21, 22) versehenen Spritzenkörpern (1, 2), welche als Doppelkammerspritzenkörper ausgebildet sind, wobei in den mündungsseitigen Kammern (25, 27) die Proteinkomponente (26) und die Thrombinkomponente (28) in jeweils lyophiliserter Form und in den anderen Kammern (29, 30) das Lösungsmittel untergebracht sind und wobei auf die Anschlußstücke (14, 15) der Spritzenkörper ein Verbindungskopf (16) aufsetzbar ist, der für jede der zu applizierenden Komponenten und gegebenenfalls für ein medizinisches Treibgas einen eigenen Förderkanal (17, 18) aufweist, wobei bei gleicher wirksamer Hubhöhe der Spritzenkörper (1, 2) der die Proteinlösung beinhaltende Spritzenkörper (1) eine um das Zwei- bis Neunfache größere Querschnittsfläche als der die Thrombinlösung beinhaltende Spritzenkörper (2) aufweist, so daß sich die Volumina der Spritzenkörper ebenfalls wie 2 : 1 bis 9 : 1 verhalten.

**Claims**

1. Arrangement for applying a tissue adhesive based on human or animal proteins, to seamlessly or seam-supportingly connect human or animal tissue or organ parts, seal wounds, stop bleedings and the like, which tissue adhesive is solidified in situ by uniting it with thrombin, which arrangement includes two syringe bodies (1, 2) ending in joining pieces and provided with commonly actuatable pistons (21, 22), which syringe bodies are designed as twin-chamber syringe bodies, wherein in the mouth-side chambers (25, 27) the protein component (26) and the thrombin component (28), each in lyophilised form, are stored, and in the other chambers (29, 30) the solvent is stored, and wherein a connecting head (16) is attachable to the joining pieces (14, 15) of the syringe bodies, which is provided with a separate conveying channel (17, 18) for each of the components to be applied and optionally for a medical propellant, wherein, with equal effective strokes of the syringe bodies (1, 2), the syringe body (1) containing the protein component has a cross sectional area that is two to nine times larger than the cross sectional area of the syringe body (2) containing the thrombin component, so that the volumes of the syringe bodies also have a ratio of 2 : 1 to 9 : 1.

## Revendications

1. Dispositif destiné à l'application d'un produit adhésif pour tissus à base de protéines humaines ou animales en vue de la réunion, sans couture ou en soutien à une couture, de parties de tissus ou d'organes humains ou animaux, de la fermeture de plaies, de l'hémostase, etc., lequel produit adhésif pour tissus est solidifié in situ par mise en contact avec la thrombine, le dispositif comportant deux corps de seringue (1, 2) munis de pistons (21, 22) actionnables collectivement et se terminant par des raccords, ayant la forme de corps de seringue à double chambre, le composant protéine (26) et le composant thrombine (28) étant logés sous une forme lyophilisée dans les chambres (25, 27) du côté embouchure et le solvant dans les autres chambres (29, 30) et une tête de liaison (16) présentant un canal de transport (17, 18) distinct pour chacun des composants à appliquer et le cas échéant pour un gaz propulseur médicinal pouvant être placée sur les raccords (14, 15) des corps de seringue, le corps de seringue (1) contenant la solution de protéine présentant, pour une même hauteur de course utile des corps de seringue (1, 2), une aire de section 2 à 9 fois supérieure à celle du corps de seringue (2) contenant la solution de thrombine, de sorte que les volumes des corps de seringue se trouvent également dans le rapport de 2 : 1 à 9 : 1.